# EUROPÄISCHE PATENTSCHRIFT

(11) **EP 0 615 754 B1**
(45) Veröffentlichungstag und Bekanntmachung des Hinweises auf die Patenterteilung: **10.07.2002**
(21) Anmeldenummer: 94103292.2
(22) Anmeldetag: 04.03.1994
(51) Int. Cl.: A61K 31/44, A61K 9/00, A61K 9/28, A61K 47/48

(54) **Feste Flupirtin enthaltende orale Darreichungsformen mit kontrollierter Wirkstoffabgabe**
Solid controlled-release administration forms containing flupertine
Formes solides d'administration orale à libération contrôlée contenant la flupertine

(30) Priorität: 18.03.1993 DE 4308572; 14.06.1993 DE 4319649
(43) Veröffentlichungstag der Anmeldung: 21.09.1994
(73) Patentinhaber: AWD.pharma GmbH & Co.KG, 01097 Dresden (DE)
(72) Erfinder: Goede, Joachim, Dr., D-63457 Hanau (DE); Hettche, Helmut, Dr., D-63128 Dietzenbach (DE); Momberger, Helmut, Dr., D-35037 Marburg (DE); Engel, Jürgen, Prof. Dr., D-63755 Alzenau (DE); Lobisch, Michael, Dr., D-64372 Ober-Ramstadt (DE)

(56) Entgegenhaltungen:
- EP-A- 0 189 788
- EP-A- 0 193 056
- EP-A- 0 207 193
- EP-A- 0 214 735
- EP-A- 0 467 164
- EP-A- 0 560 092
- Gullikson et al., Gastroenterology, 1977, 73 (3), p. 501-511

## Beschreibung

Arzneimittelzubereitungen mit kontrollierter Wirkstofffreigabe enthaltend flupirtin unter Verwendung einer Retardkomponente, wobei auf einen Gewichts-Teil Flupirtin (bezogen auf die Base) 0,001 bis 20 Teile Retardkomponente kommen und das Flupirtin auch in Form seiner physiologisch verträglichen Salze vorliegen kann und die Freisetzungsgeschwindigkeit von Flupirtin zwischen 5 und 300 mg pro Stunde beträgt, gegebenenfalls unter Zugabe eines schnell freisetzenden Anteils von Flupirtin oder eines seiner Salze.

Aufgabe der Erfindung ist es, eine feste Arzneimittelzubereitung mit dem Wirkstoff Flupirtin und ein Verfahren zu ihrer Herstellung zu schaffen, wobei die sedierenden Nebenwirkungen des Flupirtins weitgehend beziehungsweise ganz unterdrückt sind. Weiter ist es vorteilhaft, den Patienten, die der Therapie durch ein Analgetikum bedürfen, eine Formulierung zur Verfügung zu stellen, die nicht mehr so häufig eingenommen werden muß. Gerade Patienten mit starken Schmerzen, zum Beispiel Tumorpatienten, empfinden die seltenere Arzneimittelgabe sowie die längere Wirkungsdauer insbesondere bei nächtlichen Schmerzen als vorteilhaft. Durch Reduzierung der täglich anzuwendenden Menge an Wirkstoff wird außerdem der Körper, insbesondere die Leber, entlastet. Die Aufgabe wird gelöst durch eine Arzneimittelzubereitung mit kontrollierter Wirkstofffreisetzung, enthaltend übliche Hilfs- und Zusatzstoffe und eine Retardkomponente, die dadurch gekennzeichnet ist, daß der Wirkstoff Flupirtin beziehungsweise seine physiologisch verträglichen Salze kontrolliert freigesetzt wird und in einer Menge von 1 Gew.-Teil Flupirtin, bezogen auf die Base, zu 0,001 bis 20 Gew.-Teilen Retardkomponente vorhanden ist und die Freisetzung von 5 bis 300 mg, beispielsweise 10 bis 100 mg oder auch 20 bis 60 mg Flupirtin/Stunde beträgt.

Diese Arzneimittelzubereitung mit kontrollierter Freisetzung des Wirkstoffs kann auch kombiniert werden mit einer zusätzlichen Menge an Flupirtin oder eines seiner physiologisch verträglichen Salze, wobei diese zusätzliche Menge ohne Zugabe von Retardkomponente formuliert und schnell freigesetzt wird, das heißt innerhalb von 10 bis 20 Minuten. Diese zusätzliche Menge wäre als Initialphase anzusehen, die einen schnellen Wirkungseintritt der Gesamt-Darreichungsform gewährleistet. Somit kann mit einer derartigen Darreichungsform eine für Schmerzmittel wünschenswerte schnell einsetzende und lang anhaltende Wirkung erzielt werden. Der Anteil der Initialphase Flupirtin im Verhältnis zum Anteil Flupirtin, der kontrolliert freigesetzt wird, verhält sich in diesem Fall wie 2 : 1 bis 1 : 9 (jeweils Gewichtsanteile).

Wenn das Flupirtin in Form eines Salzes vorliegt, erhöht sich die vorstehend angegebene, sich auf die Base beziehende Flupirtinmenge durch das höhere Molekulargewicht des Salzes entsprechend. Die Mengenangaben in der Anmeldung bei Angabe 'Flupirtin' beziehen sich stets auf die Base und sind bei Vorliegen eines Salzes entsprechend dem erhöhten Molekulargewicht umzurechnen.

Die Aufgabe schließt auch ein Verfahren ein zur Herstellung einer Arzneimittelzubereitung mit kontrollierter Wirkstofffreigabe durch Einbringen eines Wirkstoffes in übliche Hilfs- und Zusatzstoffe und eine Retardkomponente, das dadurch gekennzeichnet ist, daß man als kontrolliert freizusetzenden Wirkstoff Flupirtin beziehungsweise seine physiologisch verträglichen Salze im Verhältnis 1 Gew.-Teil Flupirtin, bezogen auf die Base, auf 0,001 bis 20 Gew.-Teile Retardkomponente verwendet und eine Freisetzungsgeschwindigkeit von 5 bis 300 mg Flupirtin/Stunde einstellt.

In den Unteransprüchen sind bevorzugte Ausführungsformen der Erfindung beschrieben. Wenn man den Wirkstoff Flupirtin in eine Darreichungsform bringt, die den Wirkstoff über einen längeren Zeitraum freisetzt (und man damit eine 'Retardierung' der Wirksubstanz durchführt), stellt man bei mit dieser Darreichungsform behandelten Personen die Nebenwirkung Müdigkeit nicht mehr fest.

Gegenstand der Erfindung sind also feste Darreichungsformen mit kontrollierter Abgabe des Wirkstoffes Flupirtin beziehungsweise von physiologisch verträglichen Salzen des Flupirtin. Geeignete Salze sind zum beispielsweise Chlorid, Maleat, D-Gluconat.

Ein weiterer Gegenstand der Erfindung ist ein Verfahren zur Herstellung von Darreichungsformen mit kontrollierter Abgabe des Wirkstoffes Flupirtin beziehungsweise von physiologisch verträglichen Salzen des Flupirtin.

Die Bestimmung der Freisetzungsgeschwindigkeit des Flupirtin in den auf Seite 1 angegebenen Grenzen wird in einer wässrigen Lösung mit den pH-Werten 1 oder 6,8 durchgeführt. Die Einstellung der pH-Werte erfolgt durch Zusatz von Säure oder durch Zusatz eines üblichen Puffers beispielsweise Phosphatpuffer. Die Methode ist in der USP XXII, 1. Januar 1990, Seiten 1578-1579 beschrieben.

Als Darreichungsformen kommen zum Beispiel in Frage: Tabletten, Filmtabletten, Hartgelatinekapseln, Weichgelatinekapseln, Pellets, Granulate, Dragees, Suppositorien, Mikrokapseln, wässrige oder ölige Suspensionen, ölige Lösungen.

Die erfindungsgemäßen Zubereitungen können wie folgend erhalten werden:
1. Durch Bindung von Flupirtin an physiologisch verträgliche Kationenaustauscher. Als solche Kationenaustauscher können zum Beispiel eingesetzt werden:
   Acryl- und Methacrylharze mit austauschbarem Proton, sauren Gruppen: COO⊖, z.B. Amberlite® IRP-64 Polystyrolharze mit austauschbarem Na, saure Gruppen: SO₃⊖, z.B. Amberlite® IRP-69
   Bei den Ionenaustauschern handelt es sich um saure Ionenaustauscher. Das Maximalverhältnis Flupirtin: Ionenaustauscher beträgt etwa 1:1, das Minimalverhältnis etwa 1 Gewichtsteil Wirkstoff auf 800 Teile Ionenaustauscherharz. Vorzugsweise werden auf 1 Gew.-Teil Wirkstoff 1 bis 400 Gew.-Teile Ionenaustauscher, ganz besonders 1 bis 100 Gew.-Teile Ionenaustauscher verwendet.
   Die Bindung des Flupirtins erfolgt, indem man eine Flupirtinlösung durch ein Bett des Ionenaustauschers in einer Säule laufen läßt oder den Ionenaustauscher in einer Lösung von Flupirtin suspendiert, nach Rühren abfiltriert und wäscht. Der beladene Ionenaustauscher wird getrocknet bei Temperaturen bis zu etwa 50°C. Vorzugsweise werden die beladenen Ionenaustauscherpartikel noch mit einer Umhüllung versehen, wie es beispielsweise in US-A-4 221 776 beschrieben ist. Ein Vorteil des zusätzlichen Einhüllens besteht darin, daß sich die Freisetzungsrate des Wirkstoffes durch die Wahl des Hüllenmaterials verändern und beeinflussen läßt. Das Trocknen der mit Hülle versehenen beladenen Ionenaustauscherpartikel kann mit Warmluft von 70°C bis 90°C erfolgen.
   Die beladenen Ionenaustauscherpartikel können in Hartgelatinekapseln gefüllt, oder mit Hilfe von Wasser und Verdickungsmitteln, Geschmacks- und Stabilisierungs- und Konservierungsstoffen als eine Suspension als Darreichungsform hergestellt werden.
2. Umhüllung von Wirkstoffteilchen, Granulat- oder Pelletkörner oder Flupirtin enthaltende Tabletten mit Überzügen aus folgenden Substanzen, wobei diese Hüllsubstanzen auch in Mischung verwendet werden können: Hydroxypropylmethylcellulosephthalat oder -acetatsuccinat; Cellulose-, Stärke-, sowie Polyvinylacetatphthalat; Carboxymethylcellulose; Polyvinylacetat; Methylcellulose-phthalat, Methylcellulose-succinat, Methylcellulose-phthalatsuccinat sowie Methylcellulose-phthalsäurehalbester; Zein; Ethylcellulose sowie Ethylcellulose-succinat; Schellack; Gluten; Ethylcarboxyethylcellulose; Ethacrylat- Maleinsäureanhydrid-Copolymer; Maleinsäureanhydrid-Vinylmethylether-Copolymer; Styrol-Maleinsäure-Copolymerisate; 2-Ethyl-hexyl-acrylat-maleinsäureanhydrid; Crotonsäure-Vinylacetat-Copolymer; Glutaminsäure/Glutaminsäureester-Copolymer; Carboxymethylethylcelluloseglycerinmonooctanoat; Celluloseacetatsuccinat; Polyarginin; Fett, Öle, Wachse, Fettalkohole; Anionische Polymerisate aus Methacrylsäure und Methacrylsäureestern (Eudragit®L, Eudragit®S); Copolymerisate aus Acryl- und Methacrylsäureestern mit einem geringen Gehalt an Trimethylammoniummethacrylat (Eudragit®RL, Eudragit RS), Copolymerisat aus Acrylsäure, Methacrylsäure sowie deren Estern (Verhältnis der freien Carboxylgruppen zu den Estergruppen z.B. 1:1) (Eudragit®L 30 D), Copolymerisat aus Acrylsäureethyl- und Methacrylsäuremethylester (Eudragit®NE 30 D).
   Die genannten Substanzen können zusätzlich übliche Weichmacher (z.B. Dibutylsebacat, Citronen- und Weinsäureester, Glycerin und Glycerinester, Phthalsäureester und ähnliche Stoffe) enthalten, außerdem den Zusatz wasserlöslicher Substanzen wie Polyethylenglykole, Polyvinylpyrrolidon, Copoly merisat aus Polyvinylpyrrolidon und Polyvinylacetat, Hydroxypropylcellulose, Hydroxypropylmethylcellulose. Auch der Zusatz von Feststoffen wie Talkum und/oder Magnesiumstearat in der Umhüllung ist möglich.
   Die Pelletkörner, Granulatkörner oder Tabletten können auch Zusätze von organischen Säuren (wie z.B. Citronensäure, Weinsäure, Malein-, Fumar-, Ascorbinsäure) eingearbeitet enthalten.
   Die Umhüllung erfolgt durch Aufsprühen von Lösungen in organischen Lösungsmitteln oder Suspensionen der genannten Substanzen in organischen Lösungsmitteln oder Wasser, wobei noch weitere Hilfsstoffe zur Optimierung deren Verarbeitbarkeit zugesetzt sein können, wie z.B. oberflächenaktive Substanzen, Pigmente.
   Das Aufsprühen erfolgt z.B. im Dragierkessel oder in perforierten Kesseln, oder im Luftsuspensionsverfahren (z.B. Glatt Wirbelschichtanlage WLSD5). Die Umhüllung kann auch durch Koagulation wässriger Dispersionen der zuvor genannten Substanzen erfolgen, indem der Wirkstoff mit der Dispersion vermischt und das Wasser durch Trocknen entfernt wird.
   Überzogene Wirkstoffteilchen und überzogene Granulate können zu Tabletten verpreßt werden, überzogene Pellets in Hartgelatinekapseln abgefüllt oder nach Zusatz weiterer Substanzen zu Tabletten verpreßt werden.
   Beim Überziehen von Wirkstoffteilchen oder Granulaten, die Wirkstoffteilchen enthalten, wird üblicherweise mehr Hüllstoff eingesetzt als bei Pellets, da die Oberfläche, die bedeckt werden muß, wesentlich größer ist als bei Pellets.
   Auf 1 Gew.-Teil Wirkstoff können 0,001 bis 20 Gew.-Teile Hüllstoff verwendet werden. Bevorzugt ist ein Gewichtsverhältnis von 1 Teil Wirkstoff und 0,005 bis 10 Gew.-Teilen Hüllmaterial, ganz besonders bevorzugt sind 0,01 bis 5 Gew.-Teile Hüllmaterial pro 1 Gew.-Teil Wirkstoff. Das Aufbringen der Hüllstoffe erfolgt bei erhöhter Temperatur, vorzugsweise im Luftstrom, Zulufttemperatur zum Beispiel 70 bis 90°C; Temperatur der Abluft zum Beispiel bis 40°C.
3. Umhüllung von Preßlingen, Tabletten, Granulaten, die Flupirtin und eine oder mehrere osmotisch aktive Substanzen (z.B. Mannit, Sorbit) enthalten mit einer semi-permeablen Membran, z.B. aus 70 bis 90 Gewichts% Celluloseacetat und Hydroxypropylmethylcellulose (30 bis 10 Gewichts%).
   Als osmotisch aktive Substanzen kommen weiterhin in Frage: organische und anorganische Verbindungen oder lösliche Stoffe, die einen osmotischen Druckgradienten gegenüber der äußeren Flüssigkeit über die semipermeable Wand erzeugen. Osmotisch wirksame Mittel oder osmotisch wirksame Verbindungen umfassen Magnesiumsulfat, Magnesiumchlorid, Natriumchlorid, Lithlumchlorid, Kaliumsulfat, Kaliumhydrogenphosphat, Harnstoff, Saccharose und ähnliches. Weitere osmotisch wirksame Mittel sind bekannt aus den US-PS 3 854 770, 4 077 407 und 4 235 236.
   Als semi-permeable Materialien, die als Polymere zur Osmose und umgekehrten Osmose bekannt sind, kommen z.B. in Frage: Cellulose-acylat, Cellulose-diacylat, Cellulose-triacylat, Cellulose-acetat, Cellulose-diacetat, Cellulose-triacetat, β-Glucan-acetat, Acetaldehyd-dimethyl-acetat, Cellulose-acetat-ethyl-carbamat, Polyamid, Polyurethan, sulfoniertes Polystyrol, Cellulose-acetat-phthalat, Cellulose-acetatmethyl-carbamat, Cellulose-acetat-succinat, Cellulose-acetat-dimethylaminoacetat, Cellulose-acetat-chloracetat, Cellulose-dipalmitat, Cellulose-dloctanoat, Cellulose-dicaprylat, Cellulose-dipentanat, Cellulose-acetat-valerat, Cellulose-acetat-p-toluolsulfonat, Cellulose-acetat-butyrat, Ethylcellulose, selektiv permeable Polymere, die gebildet worden sind durch gemeinsame Ausfällung eines Polykations und eines Polyanions wie in den US- 3,173,876, 3,276,586, 3,541,005, 3,541,006 und 3,546,142 angegeben. Derartige Einhüllungen in semipereable Membranen können z.B. auch gemäß DE-A 33 10 081 oder DE-A-33 10 096 erfolgen.
   Der Anteil an osmotisch aktiver Substanz kann, bezogen auf 1 Gew.-Teil Flupirtin, von 1 bis 20 Gew.-Teilen, vorzugsweise 2 bis 6, ganz besonders bevorzugt 4 bis 5 Gew.-Teilen betragen. Die Hüllstoffe werden in einer Menge aufgebracht, daß die semipermeable Membran 50 bis 500 µm, vorzugsweise 100 bis 300 µm dick ist.
   Das Verarbeiten des Wirkstoffes und der osmotisch aktiven Substanzen kann zwischen Raumtemperatur und 80°C erfolgen. Zur Einstellung der Freisetzungsrate wird zum Beispiel bei 70 - 90°C Zulufttemperatur getrocknet.
   Gegebenenfalls kann die semipermeable Membran auch noch eine mikroporöse Schicht enthalten beziehungsweise können mikroporöse Stoffe eingearbeitet sein (siehe hierzu Deutsche Offenlegungsschrift 33 10 081, zum Beispiel Seite 7 - 17).
   Materialien, die zur Herstellung der mikroporösen Schicht geeignet sind, umfassen zum Beispiel Polycarbonate aus linearen Polyestern der Kohlensäure, in denen Carbonatgruppen in der Polymerkette wiederkehren, mikroporöse Materialien, die hergestellt worden sind durch Phosgenierung einer dihydroxyaromatischen Verbindung wie Bisphenol, einem mikroporösen Polyvinylchlorid, mikroporösen Polyamiden wie Polyhexamethyl-adipinsäureamid, mikroporöse Modacryl-polymere einschließlich solchen, die gebildet worden sind aus Polyvinylchlorid und Acrylnitril, mikroporöse Styrol-Acrylmonomer und deren Copolymere, poröse Polysulfone charakterisiert durch Diphenylensulfon in einer linearen Kette, halogeniertes oder eines Anhydrids mit einem Alkylenpolyol, Polyalkylsulfide, phenolische Polymere, Polyester, mikroporöse Polysaccharide mit substituierten Anhydroglucoseeinheiten, die eine abnehmende Durchlässigkeit für Wasser und biologische Flüssigkeiten besitzen, asymmetrische poröse Polymere, vernetzte Olefinpolymere, hydrophobe oder hydrophile mikroporöse Homopolymere, Copolymere oder Interpolymere mit einer verringerten Dichte sowie Materialen, die beschrieben sind in den US-PS 3 595 752, 3 643 178, 3 654 066, 3 709 774, 3 718 532, 3 803 601, 3 852 224, 3 852 388 und 3 853 601; in der GB-PS 1 126 849 und in Chemical Abstracts Band 71, 427F, 22573F, 1969.
   Weitere mikroporöse Materialien zur Herstellung der mikroporösen Schicht umfassen Polyurethane, vernetzte kettenverlängerte Polyurethane, Polyimide, Polybenzimidazole, Collodium, regenerierte Proteine, halbfestes vernetztes Polyvinyl-pyrrolidon, mikroporöse Materialien, die hergestellt worden sind durch Diffusion von mehrwertigen Kationen in Polyelektrolytsole, mikroporöse Derivate von Polystyrol wie Natriumpolystyrol-sulfonat, Polyvinylbenzyl-trimethyl-ammonium-chlorid, mikroporöse Cellulose-acylate und ähnlich mikroporöse Polymere sind bekannt aus den US-PS 3 524 753, 3 565 259, 3 276 589, 3 541 055, 3 541 006, 3 546 142, 3 615 024, 3 646 178 und 3 852 224.
   Die zur Herstellung der mikroporösen Schicht geeigneten Porenbildner umfassen Feststoffe und porenbildende Flüssigkeiten. Der Ausdruck Porenbildner, wie er hier verwendet wird, umfaßt auch Substanzen, die Mikrodurchgänge bilden und die Entfernung der Porenbildner kann zu beiden Arten führen. Der Ausdruck porenbildende Flüssigkeiten umfaßt im Rahmen dieser Beschreibung halbfeste und viskose Flüssigkeiten. Die Porenbildner können anorganisch oder organisch sein und das schichtbildende Polymer enthält im allgemeinen 5 bis 70 Gewichts% Porenbildner, insbesondere 20 bis 50 Gewichts%. Der Ausdruck Porenbildner sowohl für Feststoffe als auch für Flüssigkeiten umfaßt Substanzen, die durch die in der Anwendungsumgebung vorhandene Flüssigkeit aus dem Vorläufer der mikroporösen Membran herausgelöst, extrahiert oder ausgelaugt werden können, unter Bildung einer wirksamen offenzelligen mikroporösen Schicht. Die porenbildenden Feststoffe haben eine Teilchengröße von ungefähr 0,1 bis 200 µm und umfassen Alkalisalze wie Lithiumcarbonat, Natriumchlorid, Natriumbromid, Kaliumchlorid, Kaliumsulfat, Kaliumphosphat, Natriumacetat, Natriumcitrat und ähnliches. Organische Verbindungen wie Saccharide einschließlich der Zucker Saccharose, Glucose, Fructose, Mannit, Mannose, Galactose, Sorbit und ähnliches. Es können auch lösliche Polymere zur Anwendung kommen wie Carbowaxe, Carbopol und ähnliches. Die Porenbildner umfassen ferner Diole, Polyole, mehrwertige Alkohole, Polyalkylenglykole, Polyglykole, Poly(α-w)-alkylendiole und ähnliches.
4. Einbettung von Flupirtin bzw. Flupirtinsalzen oder Bindung an folgende Substanzen oder Mischungen dieser Substanzen:
   - Verdauliche Fette, z.B. Triglyceride von gesättigten Fettsäuren C₈H₁₆O₂ bis C₁₈H₃₆O₂ und deren Gemische, Erdnußöl und hydriertes Erdnußöl, Ricinusöl und hydriertes Ricinusöl, Olivenöl, Sesamöl, Baumwollsamenöl und hydriertes Baumwollsamenöl, Maisöl, Weizenkeimöl, Sonnenblumensamenöl, Kabeljau-Leberöl, Gemische von Mono-, Di-, Triestern der Palmitin- und Stearinsäure mit Glycerin, Glycerintrioleat, Diglykolstearat, Stearinsäure, Metallsalze von Fettsäuren, insbesondere Erdalkalimetallsalze von Fettsäuren, beispielsweise Magnesiumstearat.
   - Unverdauliche Fette bzw. fettähnliche Substanzen, z.B. Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome), Carnaubawachs, Bienenwachs, Fettalkohole (geradkettig oder verzweigtkettig) der Kettenlänge C₈H₁₇OH bis C₃₀H₆₁OH, insbesondere C₁₂H₂₅OH bis C₂₄H₄₉OH.
   - Polymere wie Polyvinylalkohol, Polyvinylchlorid, Polyacrylsäure (Carbopol®); anionische Polymerisate aus Methacrylsäure und Methacrylsäureestern (Eudragit®L, Eudragit®S), Acryl- und Methacrylsäureester-Copolymerisate mit Trimethylammoniummethacrylat (Eudragit®RL, Eudragit®RS) Copolymerisat von Acrylsäureethyl- und Methacrylsäuremethylestern (Eudragit®NE 30 D), sowie aus Acrylsäure, Methacrylsäure sowie deren Estern (Verhältnis der freien Carboxylgruppen zu den Estergruppen 1:1) (Eudragit® - L 30 D), Polyethylen, Polyglycolsäure, Polyhydroxybuttersäure, Polymilchsäure, Copolymere aus Milchsäure und Glycolsäure (Hersteller: Boehringer Ingelheim), Copolymere aus Milchsäure und Ethylenoxid, Copolymere aus Glycolsäure und Ethylenoxid, Copolymere aus Milchsäure und Hydroxybuttersäure, Hydroxypropylmethylcellulose-phthalat oder acetatsuccinat; Celluloseacetatphthalat, Stärkeacetatphthalat, sowie Polyvinylacetatphthalat; Carboxymethylcellulose; Methylcellulose-phthalat,-succinat, -phthalatsuccinat, Methylcellulose-phthalsäurehalbester; Zein; Ethylcellulose; Schellack, Gluten; Ethylcarboxyethylcellulose; Ethacrylat-Maleinsäureanhydrid-Copolymer; Maleinsäureanhydrid-Vinylmethylehter-Copolymer; Styrol-Maleinsäure-Copolymerisate; 2-Ethylhexyl-acrylat-maleinsäureanhydrid; Crotonsäure-Vinylacetat-Copolymer; Glutaminsäure/Glutaminsäureester-Copolymer; Carboxymethylcellulose-glycerinmonooctanoat; Celluloseacetatsuccinat; Polyarginin; quervernetztes Alginat; quervernetzte Gelatine;
   - Quellstoffe wie Methylcellulose, Hydroxypropylcellulose, Hydroxypropylmethylcellulose (Pharmacoat, Methocel E = Propylenglykoläther der Methylcellulose), Alginsäure und ihre Salze (Na-, Ca-Salz, auch Mischungen aus Natriumalginat und Calciumsalzen zum Beispiel CaHPO₄), Stärke, Carboxymethylstärke, Carboxymethylcellulose und deren Salze (zum Beispiel Na-Salz), Galaktomannan, Gummi arabicum, Karaya Gummi, Ghatti Gum, Agar-agar, Carrageen, Xanthan-Gummi, Guar Gummi und seine Derivate, Johannisbrotkernmehl, Propylenglykolalginat, Pektin, Traganth.

Auf 1 Gewichtsteil Flupirtin werden bei diesen Retardkomponenten 0,001 bis 20 Gew.-Teile Retardkomponente, vorzugsweise 0,5 bis 10 Gew.-Teile, ganz besonders bevorzugt 1,0 bis 5 Gew.-Teile verwendet. Die Herstellung dieser Zubereitungen erfolgt bei Temperaturen zwischen 18°C und 80°C.

Die Herstellung dieser Darreichungsform kann erfolgen:
a) durch Lösen oder Dispergieren von Flupirtin oder seinen Salzen in den genannten Fetten oder fettähnlichen Substanzen oder Mischungen davon, auch unter Schmelzen der genannten Substanzen und anschließend Wiederabkühlen, Zerkleinern, evtl. Zufügen weiterer Substanzen wie z.B. die oben erwähnten wasserlöslichen oder in Wasser quellbaren Substanzen und Verpressung zu Tabletten. Das Abkühlen der Schmelze und Zerkleinern kann auch in einem Schritt zusammengefasst werden, indem die Schmelze in kaltem Wasser dispergiert wird oder einer Sprüherstarrung unterworfen wird. Bei Verwendung der obengenannten Öle als Retardierungsmittel wird Flupirtin bzw. dessen Salz im Öl gelöst oder suspendiert und, evtl. nach Zusatz von bis zu 2 % Aluminiummonostearat, in Ampullen abgefüllt und nachfolgend sterilisiert oder, evtl. nach Zusatz von Geschmacksstoffen und/oder Sedimentationsverzögerern, wie hochdisperses Siliciumdioxid (z.B. Aerosil®) homogenisiert und in Flaschen gefüllt,
b) durch Mischen von Flupirtin oder seinen Salzen mit den genannten Fetten, Polymeren oder Quellstoffen oder Mischungen dieser Substanzen, auch unter Anwendung von Wärme, und Verpressen der Mischungen, evtl. nach Zusatz weiterer Hilfsstoffe zu Tabletten oder Formung zu Pellets,
c) durch Mischen von Flupirtin oder seinen Salzen mit Lösungen der genannten Fette oder Polymeren in Wasser oder organischen Lösungsmitteln, wie z.B. Ethanol, Ethylacetat, Aceton oder Isopropanol, evtl. Mischen mit Trägermaterialien wie Cellulosen, sowie nachfolgendes Abdampfen der Lösungsmittel und Vermischen der erhaltenen Wirkstoffeinbettung mit weiteren Hilfstoffen und Verarbeitung zu Formkörpern, wie z.B. Tabletten oder Pellets.
d) durch Anfeuchten einer Mischung aus Flupirtin oder seinen Salzen und den genannten Quellstoffen mit organischen Lösungsmitteln wie Ethanol, Ethylacetat, Aceton oder Isopropanol, evtl. unter Beifügung von Bindemitteln, wie Polyvinylpyrrolidon oder Copolymeren aus Polyvinylpyrrolidon und Polyvinylacetat, Granulieren der erhaltenen Mischung, nachfolgendes Trocknen, Zusatz von evtl. weiteren Hilfsstoffen und Pressung der Mischung zu Tabletten,
e) durch Mischen von Flupirtin oder seinen Salzen mit einer Lösung von Natur- oder Kunstharzen, wie Schellack oder Polyvinylacetat in Polyethylenglykol vom Molgewicht 200 bis 1500, evtl. Zusatz weiterer Hilfsstoffe, wie z.B. Stearate oder Quellstoffe und Verkapseln der erhaltenen Masse in Weich- oder Hartgelatinekapseln.

Ganz allgemein erfolgt die Herstellung der Arzneimittelzubereitungen in an sich bekannter Weise, wobei neben den Retardkomponenten die bekannten und üblichen pharmazeutischen Hilfsstoffe sowie neben den Retardkomponenten die bekannten und üblichen pharmazeutischen Hilfsstoffe sowie sonstige übliche Träger- und Verdünnungsmittel verwendet werden können, wobei die als Retardkomponente genannten Hilfsstoffe auch noch andere Funktionen ausüben können, z.B. als Formtrennmittel oder als Sprengmittel wirken.
Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u. f., H.v. Czetsch-Lindenwald, Hilfsstoffe für Pharmazie und angrenzende Gebiete; Pharm. Ind., Heft 2, 1961, Seite 72 u. ff.; Dr. H.P. Fiedler, Lexikon der Hilfsstoffe für Pharmazie, Kosmetik und angrenzende Gebiete, 3. Auflage, Editio Cantor, Aulendorf in Württemberg.

Beispiele für übliche Hilfsstoffe, Trägerstoffe und Verdünnungsmittel sind Gelatine, natürliche Zucker, wie Rohrzucker oder Milchzucker, Lecithin, Pektin, Stärke (z.B. Maisstärke) sowie Stärkederivate, Cyclodextrine und Cyclodextrinderivate, Polyvinylpyrrolidon, Gelatine, Gummi arabicum, Alginsäure, Tylose, Talkum, Lycopodium, Kieselsäure (z.B. kolloidale), Lävulose, Traganth, Natriumchlorid, Stearate, Magnesium- und Calciumsalze von Fettsäuren mit 12 bis 22 C-Atomen, insbesondere der gesättigten (z.B. Stearate), Polyethylenglycol mit einem mittlerern Molekulargewicht zwischen 200 und 20.000, vorzugsweise zwischen 200 und 5.000, insbesondere zwischen 200 und 1000, oder deren Gemische und/oder Polymerisate aus Vinylpyrrolidon und/oder Mischpolymerisate aus Vinylpyrrolidon und Vinylacetat. Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglycol, Pentaerythrit, Sorbit, Mannit usw., die gegebenenfalls auch verethert sein können, Benzylbenzoat, Dioxolane, Glyzerinformale, Tetrahydrofurfurylalkohol, Polyglykolether mit C₁ bis C₁₂-Alkoholen, Dimethylacetamid, Lactamide, Lactate, Ethylcarbonate, Silicone (insbesonder mittelviskose Polydimethylsiloxane), Calciumcarbonat, Natriumcarbonat, Calciumphosphat, Natriumphosphat, Magnesiumcarbonat, Gummi arabicum, Alginsäure, Stearate, Fette und ähnlich wirkende Stoffe.

Daneben können die Darreichungsformen grenzflächenaktive Substanzen enthalten. Als Beispiel seien genannt: Alkaliseifen, wie Alkalisalze höherer Fettsäuren (z.B. Na-palmitat, Na-stearat) oder deren Derivate (z.B.Na-rizionlatschwefelsäureester); sulfurierte Verbindungen oder sulfonierte Verbindungen, die durch Umsetzung von höheren Fettalkoholen mit Schwefelsäure oder Chlorsulfonsäure entstehen und z.B. als Natriumsalze eingesetzt werden (z.B. Natriumlaurylsulfat, Natriumcetylsulfat, Natriumstearylsulfat, Natriumcetylsulfonat); Salze der Gallensäuren; Saponine; quartäre Ammoniumverbindungen; Partialfettsäureester des Sorbitans; Partialfettsäureester und Fettsäureester des Polyoxyethylensorbitans; Sorbitolether des Polyoxyethylens; Fettsäureester des Polyoxyethylens; Fettalkoholether des Polyoxyethylens; Fettsäureester der Saccarose; Fettsäureester des Polyglycerols; Proteine; Lecithine.

Die Darreichungsformen können auch Cellulosen enthalten, insbesondere wenn Komprimate hergestellt werden sollen. Als solche kommen in Frage: gereinigte Cellulose (z.B. als Elcema® im Handel) oder mikrokristalline Cellulose, wie sie z.B. unter dem Namen Avicel® im Handel ist. Aber auch andere Füllmittel mit Bindemittelfunktion, wie Calciumhydrogenphosphat, Milchzucker, Stärken (z.B. Kartoffelstärke, Maisstärke, modifizierte Stärken wie Starch ST 1500/Colorcon), Glucose, Mannit, Saccharose können eingesetzt werden. Die Darreichungsformen können darüber hinaus Sedimentationsverzögerer enthalten, wie z.B. hochdisperse Kieselsäuren, die eine Oberfläche von 50 bis 500 m²/g, insbesondere 100 bis 400 m²/g aufweisen (bestimmt nach der BET-Methode). Diese sind im Handel z.B. unter dem Namen Aerosil® erhältlich.

Außerdem kann der Einsatz von Formtrennmitteln in der Darreichungsform sinnvoll sein. Als solche wären zu nennen:
Talk oder silikonisierter Talk, Calcium- und Magnesiumstearat, Stearinsäure, Paraffin, hydrierte Fette und Öle, Siliconölemulsion.

Als weitere Hilfsstoffen auch Stoffe in Frage, die den Zerfall bewirken (sogenannte Sprengmittel) wie: quervernetztes Polyvinylpyrrolidon, Natriumcarboxymethylstärke und Ultraamylopektin.

Zur Herstellung von Lösungen und Suspensionen kommen beispielsweise Wasser oder physiologisch verträgliche organische Lösungsmittel in Frage, wie z.B. Ethanol, 1,2-Propylenglykol, Polyglykole und deren Derivate. Für injizierbare Lösungen oder Suspensionen kommen zum Beispiel nicht-toxische parenteral verträgliche Verdünnungsmittel oder Lösungsmittel in Frage, wie z.B.: Wasser, 1,3-Butandiol, Ethanol, 1,2-Propylenglykol, Polyglykole in Mischung mit Wasser, Ringer's Lösung, isotonische Kochsalzlösung.

Darüberhinaus ist der Zusatz von Stabilisatoren, Farbstoffen, Antioxidatien und Komplexbildnern (z.B. Ethylendiaminotetraessigsäure) und dergleichen möglich sowie den Zusatz von Säuren wie Citronensäure, Weinsäure, Maleinsäure, Fumarsäure.

Als Antioxidantien kommen beispielsweise Natriummethabisulfit, Cystein, Ascorbinsäure und deren Ester (z.B. -palmitat), Flavonoide, Gallussäure, Gallussäure-alkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Citronensäure, Phosphorsäure) zur Anwendung.

Als Konservierungsmittel kommen beispielsweise Sorbinsäure, p-Hydroxybenzoesäureester (z.B. Niederalkylester), Benzoesäure, Natriumbenzoat, Trichlorisobutylalkohol, Phenol, Kresol, Benzethoniumchlorid und Formalinderivate in Betracht.

Als Plastifizierungsmittel für Hüllstoffe können in Frage kommen:
Citronen- und Weinsäureester (Acetyltriethyl-, Acetyl-tributyl-, Tributyl-, Triethyl-citrat); Glycerin und Glycerinester (Glycerindiacetat, - triacetat, acetylierte Monoglyceride, Rizinusöl); Phthalsäureester (Dibutyl-, Diamyl-, Diethyl-, Dimethyl-, Dipropyl-, D-(2-Methoxy- oder ethoxyehtyl)-phthalat, Ethylphthalyl- und Butylphthalylethyl- und butylglycolat); Alkohole (Propylenglykol, Polyethylenglykol verschiedener Kettenlängen), Adipate (Diethyl-, Di(1-Methoxy- oder ethoxyethyl)adipat); Benzophenon; Diethyl- und Dibutylsebacat, -succinat, -tartrat; Diethylenglykoldipropionat; Ethylenglykoldiacetat, - dibutyrat, -dipropionat; Tributylphosphat, Tributyrin; Polyethylenglykolsorbitanmonooleat; Sorbitanmonooleat.

Zum Aufbringen der Retardkomponenten bzw. Hüllstoffe können Lösungsmittel verwendet werden aus der Gruppe der wässrigen Lösungsmittel, Alkohole, Ketone, Ester, Ether, aliphatischen Kohlenstoffe, halogenierten Lösungsmittel sind unter anderem Aceton, Diacetonalkohol, Methanol, Ethanol, Isopropylalkohol, Butylalkohol, Methylacetat, Ethylacetat, Isopropylace tat. n-Butylacetat, Methylisosbutylketon, Methylpropylketon, n-Hexan, Ethyl-glykol-monoethyl-ether, Ethylen-glykol-monoethylacetat, Methylendichlorid, Ehtyl-dichlorid, Propylendichlorid, Tetrachlorkohlenstoff, Nitroethan, Nitropropan, Tetrachorethan, Ethylether, Isopropylether, Cyclophexan, Cyclooctan, Benzol, Toluol, Naphtha, 1,4-Dioxan, Tetrahydrofuran, Diethylenglykoldimethylether, Wasser und deren Gemische wie Aceton und Wasser, Aceton und Methanol, Aceton und Ethylalkohol, Methylendichorid und Methanol und Ethylendichlorid und Methanol sowie deren Gemische. Im Laufe des Umhüllungsprozesses werden diese Lösungsmittel wieder entfernt. Unabhängig vom Herstellverfahren sind die erfindungsgemäßen Darreichungsformen dadurch gekennzeichnet, daß sie den Wirkstoff Flupirtin oder seine physiologisch verträglichen Salze mit einer Freisetzungsgeschwindigkeit zwischen 5 und 300 mg pro Stunde an Körperflüssigkeiten abgeben bzw. in diese übertreten lassen.

Dosierungsangaben beziehen sich immer auf Flupirtin als Base; werden Salze des Flupirtins eingesetzt, so ist entsprechend dem Molgewicht umzurechnen.

Die Gehalte an Flupirtin in den erfindungsgemäßen Zubereitungen betragen
10 mg - 3000 mg, vorzugsweise
20 mg - 2000 mg
50 mg - 1500 mg

Die genannten Einzeldosen können 1- bis 5 mal, vorzugsweise 1- bis 3mal, insbesondere 1- bis 2mal täglich angewendet werden.

Im allgemeinen wird eine Freisetzungsgeschwindigkeit aus einer Tablette oder Kapsel von 20 - 30 mg Flupirtin pro Stunde angestrebt.

### Beispiel 1

### Tabletten mit 200 mg Flupirtinmaleat

40,0 kg Flupirtinmaleat werden mit 30,0 kg Polyacrylsäure (Handelsname: Carbopol® 934, Goodrich), 18 kg mikrokristalliner Cellulose, 5 kg Lactose-Monohydrat und 5 kg Citronensäure gemischt. Die Mischung wird mit 30,0 kg gereinigtem Wasser durchfeuchtet. Nach Trocknung der Masse auf eine relative Feuchte von 20-30 % wurde die Mischung gemahlen und durch ein Sieb der Maschenweite 0,8 mm gesiebt. Anschließend werden 7,0 kg mikrokristalline Cellulose, 3,4 kg Lactose Monohydrat, 0,6 kg Hochdisperses Siliciumdioxid und 1,0 kg Magnesiumstearat dazugemsicht. Die Mischung wird zu Tabletten von 550 mg Gewicht, einem Druchmesser von 10 mm und einem Wölbungsradius von 8 mm gepreßt.

Die Tabletten können gegebenenfalls mit einem magensaftlöslichen oder magensaftpermeablen Filmüberzug versehen werden.

Eine Tablette enthält 200 mg Flupirtinmaleat. Die Freisetzung wird bestimmt mit der Apparatur 2 der USP XXII (Freisetzungsmedium 500 ml 0,1 N HCl oder Phosphat-Puffer pH 6.8, Umdrehungsgeschwindigkeit des Paddles: 90 UpM). Sie beträgt:

| | | | Freisetzungsmedium: |
|---|---|---|---|
| Nach | 1 Stunde | 19 % | |
| | 2 Stunden | 51 % | 0,1 N HCl |
| | | | |
| | 3 Stunden | 75 % | |
| | 4 Stunden | 88 % | Phosphat-Puffer |
| | 5 Stunden | 92 % | pH 6.8 |

### Beispiel 2

### Kapseln mit 200 mg Flupirtinmaleat in Form von lackierten Pellets

1000 g Flupirtinmaleat werden mit 20 g Hochdispersem Siliciumdioxid gemischt. Die Pulvermischung wird auf 1000 g Neutralpellets vom Durchmesser 0,6 - 0,85 mm aufgebracht. Hierzu werden die Neutralpellets mit dem Teil einer Lösung von 50 g eines Copolymerisats mit anionischem Charakter auf Bais von Methacrylsäure und Methylmethacrylat (Handelsname: Eudragit L 100 / Röhm Pharma, Weiterstadt) sowie 50 g Schellack in 900 g Ethanol 96 % befeuchtet und anschließend Pulvermischung aufgetragen. Nach dem Trocknen wird dieser Arbeitsgang so oft wiederholt, bis die Pulvermischung völlig aufgetragen ist.

Zur Weiterverarbeitung wird die Fraktion zwischen 0,8 und 1,25 mm Pelletdurchmesser eingesetzt.

Auf 400 g der so hergestellten Pellets werden eine Lösung aus 0,8 g eines Copolymerisats mit anionischem Charakter auf Basis von Methacrylsäure und Methylmethacrylat (Handelsname: Eudragit L 100 / Röhm Pharma, Weiterstadt) und 0,8 g Schellack in 14,4 g Ethanol 96 % und 16 g Talkum nach dem oben beschriebenen Verfahren aufgetragen.

Die erhaltenen Pellets werden zu 442,7 mg in Hartgelatinekapseln abgefüllt. Eine Kapsel enthält 200 mg Flupirtinmaleat.

Die Freisetzung wird bestimmt wie in Beispiel 1. Sie beträgt:

| | | | Freisetzungsmedium: |
|---|---|---|---|
| Nach | 1 Stunde | 43 % | |
| | 2 Stunden | 56 % | 0,1 N HCl |
| | | | |
| | 4 Stunden | 69 % | Phosphat- |
| | 6 Stunden | 85 % | puffer |
| | 8 Stunden | 95 % | pH 6,8 |

### Beispiel 3:

### Hartgelatinekapseln mit 200 mg Flupirtinmaleat, davon 70 mg schnell freisetzend und 130 mg verzögert freisetzend

470 g Flupirtinmaleat werden mit einer Lösung aus 9 g Copolyvidon (Handelsname: Kollidon VA 64 /BASF) in 110 g gereinigtem Wasser angefeuchtet. Es wird mit so viel Wasser nachgefeuchtet, bis eine granulierfähige Masse entsteht. Diese Masse wird durch ein Sieb der Maschenweite 3 mm gepreßt und anschließend getrocknet. Nach dem Sieben durch ein Sieb der Maschenweite 1 mm wird das Granulat mit 5 g Magnesiumstearat und 2,5 g Hochdispersem Siliciumdioxid gemischt und zu 72,5 mg in Hartgelatinekapseln gefüllt. Zusätzlich werden in diese Hartgelatinekapseln 287,8 mg der Pellets aus Beispiel 2 gefüllt.

### Beispiel 4

### Zweischichttabletten mit 100 mg Flupirtinmaleat, davon 50 mg schnell freisetzend und 50 mg verzögert freisetzend

10 kg Flupirtinmaleat werden mit 10 kg Calciumhydrogenphosphat gemischt und mit einer wässrigen Lösung von 1 kg Polyvinylpyrrolidon (Kollidon 25/BASF) in 8 kg gereinigtem Wasser angefeuchtet.
Es wird mit soviel Wasser nachgefeuchtet, bis eine granulierfähige Masse entsteht. Die Masse wird durch ein Sieb der Maschenweite 3 mm gegeben und getrocknet. Nach dem Sieben durch ein Sieb der Maschenweite 1 mm werden 6,95 kg Maisstärke, 5 kg mikrokristalline Cellulose, 0,25 kg hochdisperses Siliciumdioxid und 0,6 kg Magnesiumstearat zugemischt.

Die erhaltene Mischung wird zusammen mit der in Beispiel erhaltenen Preßmischung zu Zweischichttabletten von Gesamtgewicht 306,5 mg gepreßt. In diesen Zweischichttabletten besteht eine Schicht aus 169 mg der oben erhaltenen Mischung, die andere Schicht aus 137,5 mg der in Beispiel 1 erhaltenen Preßmischung. Dadurch enthält die Zweischichttablette 100 mg Flupirtinmaleat, davon 50 mg schnell freisetzend und 50 mg verzögert freisetzend.

Die Zweischichttabletten können nach üblichen Verfahren mit einem magensaftlöslichen oder magensaftpermeablen Filmüberzug versehen werden.

## Patentansprüche

1. Arzneimittelzubereitungen mit kontrollierter Wirkstoffabgabe enthaltend Flupirtin oder eines seiner physiologisch verträglichen Salze, wobei der Wirkstoff gegebenenfalls unter Zusatz von Trägerstoffen, Cellulosen, Verdünnungs-, Binde-, Formtrenn-, Spreng- und/oder Konservierungsmitteln, Stabilisatoren, Sedimentationsverzögerern und/oder Antioxidantien in ein oder mehreren Retardkomponenten eingebettet ist, wobei als Retardkomponenten verdauliche und unverdauliche Fette, Polymere und/oder Quellstoffe eingesetzt werden, wobei auf ein Gewichtsteil Flupirtin (bezogen auf die Base) 0,001 bis 20 Teile Retardkomponente kommen und die Freisetzungsgeschwindigkeit von Flupirtin zwischen 5 und 300 mg pro Stunde beträgt, bestimmt nach der Methode der USP XXII mit Apparat 2 in einer wässrigen Prüflösung von pH 1,0 und/oder pH 6,8.

2. Arzneimittelzubereitungen mit kontrollierter Wirkstoffabgabe enthaltend Flupirtin oder eines seiner physiologisch verträglichen Salze, wobei der Wirkstoff gegebenenfalls unter Zusatz von Weichmachern, Feststoffen wie Talkum und/oder Magnesiumstearat, Polyethylenglykolen, Polyvinylpyrrolidon, Copolymerisat aus Polyvinylpyrrolidon und Polyvinylacetat, Hydroxypropylcellulose, Hydroxymethylcellulose und/oder organischen Säuren mit ein oder mehreren Retardkomponenten umhüllt ist, wobei als Retardkomponenten Ethylcellulosen, Schellacke, Polymerisate aus Methacrylsäure und Methacrylsäureestern wie Eudragit®L, Eudragit®S; Copolymerisate aus Acryl- und Methacrylsäureestern mit einem geringen Gehalt an Trimethylammoniummethacrylat wie Eudragit®RL, Eudragit®RS, Copolymerisat aus Acrylsäure, Methacrylsäure sowie deren Estern (Verhältnis der freien Carboxylgruppen zu den Estergruppen z. B. 1:1) wie Eudragit®L 30 D und Copolymerisat aus Acrylsäureethyl- und Methacrylsäuremethylester wie Eudragit®NE 30 D eingesetzt werden, wobei auf ein Gewichtsteil Flupirtin (bezogen auf die Base) 0,001 bis 20 Teile Retardkomponente kommen und die Freisetzungsgeschwindigkeit von Flupirtin zwischen 5 und 300 mg pro Stunde beträgt, bestimmt nach der Methode der USP XXII mit Apparat 2 in einer wässrigen Prüflösung von pH 1,0 und/oder pH 6,8.

3. Arzneimittelzubereitungen nach Anspruch 1 oder 2 **dadurch gekennzeichnet, daß** die Darreichungsformen für die orale Anwendung 50 bis 600 mg, für die parenterale Anwendung 50 bis 500 mg und für die dermale Anwendung 5 bis 5000 mg Flupirtin-Wirkstoff enthalten.

4. Arzneimittelzubereitungen nach Anspruch 1 **dadurch gekennzeichnet, daß** vorzugsweise 1 Gewichtsteil Flupirtin-Wirkstoff in 0,5 bis 5 Gewichtsteilen Retardkomponenten eingebettet ist.

5. Arzneimittelzubereitungen nach Anspruch 2 **dadurch gekennzeichnet, daß** vorzugsweise 1 Gewichtsteil Flupirtin-Wirkstoff mit 0,01 bis 5 Gewichtsteilen Retardkomponenten umhüllt ist.

6. Arzneimittelzubereitungen nach Anspruch 1 **dadurch gekennzeichnet, daß** als verdauliche Fette hydriertes Erdnuß- oder Ricinusöl eingesetzt werden.

7. Arzneimittelzubereitungen nach Anspruch 1 **dadurch gekennzeichnet, daß** als unverdauliche Fette Ester von aliphatischen gesättigten oder ungesättigten Fettsäuren mit 2 bis 22 C-Atomen mit einwertigen aliphatischen Alkoholen mit 1-20 C-Atomen, Carnauwachs, Bienenwachs oder Fettalkohole mit 8 bis 30 C-Atomen eingesetzt werden.

8. Arzneimittelzubereitungen nach Anspruch 1 **dadurch gekennzeichnet, daß** als Polymere Polyvinylakohol, Polyacrylsäure, anionische Polymerisate aus Methacrylsäure und Methacrylsäureestern (Eudragit®L, Eudragit®S), Acryl- und Methacrylsäureester-Copolymerisate mit Trimethylammoniummethacrylat (Eudragit®RL, Eudragit® RS), Copolymerisat aus Acrylsäureethyl- und Methacrylsäuremethylester (Eudragit®NE 30 D) sowie Copolymerisat aus Acrylsäure, Methacrylsäure sowie deren Estern (Verhältnis der freien Carboxylgruppen zu den Estergruppen 1:1) (Eudragit®L 30 D) eingesetzt werden.

9. Arzneimittelzubereitungen nach Anspruch 1 **dadurch gekennzeichnet, daß** als Quellstoffe Methylcellulosen, Hydroxypropylcellulosen, Hydroxypropylmethylcellulosen, Alginsäure und ihre Salze, auch Mischungen aus Natriumalginat und Calziumsalzen, Stärke, Carboxymethylstärke, Carboxymethylcellulose und deren Salze, Galaktomannan, Gummi arabicum, Karaya Gummi, Ghatti Gum, Agar-agar, Carrageen, Xanthan-Gummi, Guar-Gummi und seine Derivate, Johannisbrotkernmehl, Propylenglykolalginat, Pektin und Traganth eingesetzt werden.

10. Verfahren zur Herstellung von Arzneimittelzubereitungen mit kontrollierter Wirkstofffreigabe, enthaltend Flupirtin oder eines seine physiologisch verträglichen Salze gemäß Anspruch 1 **dadurch gekennzeichnet, daß** man ein Gewichtsteil Flupirtin (bezogen auf die Base) mit 0,001 bis 20 Teilen einer Retardkomponente und gegebenenfalls unter Zusatz von Trägerstoffen, Cellulosen, Verdünnungs-, Binde-, Formtrenn-, Spreng- und/oder Konservierungsmitteln, Stabilisatoren, Sedimentationsverzögerern und/oder Antioxidantien verarbeitet und eine Freisetzungsgeschwindigkeit von Flupirtin zwischen 5 und 300 mg pro Stunde einstellt, bestimmt nach der Methode der USP XXII mit Apparat 2 in einer wässrigen Prüflösung von pH 1,0 und/oder pH 6,8.

11. Verfahren zur Herstellung von Arzneimittelzubereitungen mit kontrollierter Wirkstofffreigabe, enthaltend Flupirtin oder eines seine physiologisch verträglichen Salze gemäß Anspruch 2 **dadurch gekennzeichnet, daß** man ein Gewichtsteil Flupirtin (bezogen auf die Base) mit 0,001 bis 20 Teilen einer Retardkomponente und gegebenenfalls unter Zusatz von Weichmachern, Feststoffen wie Talkum und/oder Magnesiumstearat, Polyethylenglykolen, Polyvinylpyrrolidon, Copolymerisat aus Polyvinylpyrrolidon und Polyvinylacetat, Hydroxypropylcellulose, Hydroxymethylcellulose und/oder organischen Säuren verarbeitet und eine Freisetzungsgeschwindigkeit von Flupirtin zwischen 5 und 300 mg pro Stunde einstellt, bestimmt nach der Methode der USP XXII mit Apparat 2 in einer wässrigen Prüflösung von pH 1,0 und/oder pH 6,8.

12. Verfahren zur Herstellung von Arzneimittelzubereitungen nach Anspruch 10 oder 11 **dadurch gekennzeichnet, daß** der Wirkstoff mit ein oder mehreren Retardkomponenten umhüllt oder in ein oder mehreren Retardkomponenten eingebettet wird.

13. Arzneimittelzubereitungen enthaltend Flupirtin oder eines seiner physiologisch verträglichen Salze **dadurch gekennzeichnet, daß** neben einem Teil mit kontrollierter Wirkstofffreigabe, erhältlich durch das Verfahren gemäß der Ansprüche 10 oder 11 ein Teil mit sofortiger Wirkstofffreigabe enthalten ist, wobei das Verhältnis im Gehalt des Flupirtin-Wirkstoffs zwischen dem Teil mit kontrollierter Wirkstofffreigabe und dem Teil mit sofortiger Wirksstofffreigabe zwischen 1:2 und 9:1 liegt.

## Claims

1. Pharmaceutical preparations in which release of the active compound is controlled and which comprise flupirtine or one of its physiologically tolerated salts, with the active compound being embedded, where appropriate in the added presence of carrier substances, cellulose, diluents, binders, mould release agents, disintegrants, preservatives, stabilizers, sedimentation retarders and/or antioxidants, in one or more retarding components, with digestible and undigestible fats, polymers and/or swelling compounds being employed as retarding components, with from 0.001 to 20 parts of retarding component being used per one part by weight of flupirtine (related to the base) and the rate of release of flupirtine being between 5 and 300 mg per hour, as determined in accordance with the USP XXII method using appliance II in an aqueous test solution of pH 1.0 and/or pH 6.8.

2. Pharmaceutical preparations in which release of the active compound is controlled and which comprise flupirtine or one of its physiologically tolerated salts, with the active compound being covered, where appropriate in the added presence of plasticizers, solids such as talc and/or magnesium stearate, polyethylene glycols, polyvinylpyrrolidone, copolymer consisting of polyvinylpyrrolidone and polyvinyl acetate, hydroxypropyl cellulose, hydroxymethyl cellulose and/or organic acids, with one or more retarding components, with ethyl celluloses, shellacs, polymers consisting of methacrylic acid and methacrylic esters, such as Eudragit® L and Eudragit® S; copolymers consisting of acrylic and methacrylic esters having having a low content of trimethylammonium methacrylate, such as Eudragit® RL and Eudragit® RS, a copolymer consisting of acrylic acid, methacrylic acid and their esters (ratio of the free carboxyl groups to the ester groups e.g. 1:1), such as Eudragit® L 30 D, and a copolymer consisting of ethyl acrylate and methyl methacrylate, such as Eudragit® NE 30 D, being employed as retarding components, with from 0.001 to 20 parts of retarding component being used per one part by weight of flupirtine (related to the base) and the rate of release of flupirtine being between 5 and 300 mg per hour, as determined by the USP XXII method using appliance 2 in an aqueous test solution of pH 1.0 and/or pH 6.8.

3. Pharmaceutical preparations according to Claim 1 or 2, **characterized in that** the administration forms comprise from 50 to 600 mg of flupirtine active compound for oral use, from 50 to 500 mg for parenteral use and from 5 to 5000 mg for dermal use.

4. Pharmaceutical preparations according to Claim 1, **characterized in that** 1 part by weight of flupirtine active compound is preferably embedded in from 0.5 to 5 parts by weight of retarding components.

5. Pharmaceutical preparations according to Claim 2, **characterized in that** 1 part by weight of flupirtine active compound is preferably covered with from 0.01 to 5 parts by weight of retarding components.

6. Pharmaceutical preparations according to Claim 1, **characterized in that** the digestible fats employed are hydrogenated groundnut oil or castor oil.

7. Pharmaceutical preparations according to Claim 1, **characterized in that** the undigestible fats employed are esters of aliphatic saturated or unsaturated fatty acids having from 2 to 22 C atoms with monohydric aliphatic alcohols having 1-20 C atoms, carnau wax, beeswax or fatty alcohols having from 8 to 30 C atoms.

8. Pharmaceutical preparations according to Claim 1, **characterized in that** the polymers employed are polyvinyl alcohol, polyacrylic acid, anionic polymers consisting of methacrylic acid and methacrylic esters (Eudragit® L and Eudragit® S), acrylic and methacrylic ester copolymers containing trimethylammonium methacrylate (Eudragit® RL and Eudragit RS), a copolymer consisting of ethyl acrylate and methyl methacrylate (Eudragit®NE 30 D) and also a copolymer consisting of acrylic acid, methacrylic acid and their esters (ratio of the free carboxyl groups to the ester groups 1:1) (Eudragit® L 30 D).

9. Pharmaceutical preparations according to Claim 1, **characterized in that** the swelling compounds employed are methyl celluloses, hydroxypropyl celluloses, hydroxypropyl methyl celluloses, alginic acid and its salts, and also mixtures consisting of sodium alginate and calcium salts, starch, carboxymethyl starch, carboxymethyl cellulose and its salts, galactomannan, gum arabic, karaya gum, ghatti gum, agar agar, carragheen, xanthan gum, guar gum and its derivatives, carob bean flour, propylene glycol alginate, pectin and tragacanth.

10. Process for producing pharmaceutical preparations in which release of the active compound is controlled and which comprise flupirtine or one of its physiologically tolerated salts according to Claim 1, **characterized in that** one part by weight of flupirtine (related to the base) is processed together with from 0.001 to 20 parts of a retarding component and, where appropriate, in the added presence of carrier substances, celluloses, diluents, binders, mould release agents, disintegrants, preservatives, stabilizers, sedimentation retarders and/or antioxidants, and the rate of release of flupirtine is adjusted to be between 5. and 300 mg per hour, as determined in accordance with the USP XXII method using appliance 2 in an aqueous test solution of pH 1.0 and/or pH 6.8.

11. Process for producing pharmaceutical preparations in which release of the active compound is controlled and which comprise flupirtine, or one of its physiologically tolerated salts, according to Claim 2, **characterized in that** one part by weight of flupirtine (related to the base) is processed together with from 0.001 to 20 parts of a retarding component and, where appropriate, in the added presence of plasticizers, solids such as talc and/or magnesium stearate, polyethylene glycols, polyvinylpyrrolidone, a copolymer consisting of polyvinylpyrrolidone and polyvinyl acetate, hydroxypropyl cellulose, hydroxymethyl cellulose and/or organic acids, and the rate of release of flupirtine is adjusted to be between 5 and 300 mg per hour, as determined in accordance with the USP XXII method using appliance 2 in an aqueous test solution of pH 1.0 and/or pH 6.8.

12. Process for producing pharmaceutical preparations according to Claim 10 or 11, **characterized in that** the active compound is covered with one or more retarding components or embedded in one or more retarding components.

13. Pharmaceutical preparations which comprise flupirtine or one of its physiologically tolerated salts, **characterized in that** in addition to a part in which release of the active compound is controlled, which part can be obtained by the process according to Claims 10 or 11, a part is present in which release of the active compound is immediate, with the ratio of the content of the flupirtine active compound between the part in which release of the active compound is controlled and the part in which release of the active compound is immediate being between 1:2 and 9:1.

## Revendications

1. Préparations médicamenteuses ayant une libération contrôlée de principe actif, contenant de la flupertine ou un de ses sels physiologiquement compatibles, dans lesquelles le principe actif est inclus dans un ou plusieurs composants retard, le cas échéant tout en ajoutant des substances de support, des celluloses, des agents diluants, liants, de démoulage, d'éclatement et/ou de conservation, des agents stabilisants, des retardateurs de sédimentation, et/ou des agents anti-oxydants, dans lesquelles comme composants retard on met en oeuvre des graisses digestibles ou non digestibles, des polymères et/ou des substances gonflantes, dans lesquelles pour une partie en poids de flupertine (rapporté à la base) on utilise de 0,001 à 20 parties de composants retard, et la vitesse de libération de la flupertine s'élève entre 5 et 300 mg par heure, déterminée selon la méthode de la pharmacopée US XXII avec l'appareillage 2, dans une solution d'essai aqueuse de pH 1,0 et/ou de pH 6,8.

2. Préparations médicamenteuses ayant une libération contrôlée de principe actif, contenant de la flupertine ou un de ses sels physiologiquement compatibles, dans lesquelles le principe actif est enrobé éventuellement en ajoutant des agents plastifiants, des matières solides comme le talc et/ou le stéarate de magnésium, des polyéthylèneglycols, de la polyvinylpyrrolidone, des produits de polymérisation à base de polyvinylpyrrolidone et d'acétate de vinyle, de l'hydroxypropylcellulose, de l'hydroxypropylméthylcellulose, et/ou des acides organiques, avec un ou plusieurs composants retard, dans lesquelles comme composant retard on met en oeuvre des éthylcelluloses, de la gomme laque, des produits de polymérisation d'acide méthacrylique et d'esters d'acide méthacrylique comme Eudragit®L, Eudragit®S, des produits de copolymérisation à partir d'acide acrylique et d'esters d'acide méthacrylique ayant une teneur faible en méthacrylate de triméthylammonium comme Eudragit®RL, Eudragit®RS, des produits de copolymérisation à partir d'acide acrylique, d'acide méthacrylique ainsi que de leurs esters (rapport des groupes carboxyliques libres aux groupes ester par exemple 1:1) comme Eudragit® L 30 D, et les produits de copolymérisation à partir d'ester éthylique d'acide acrylique et d'ester méthylique d'acide méthacrylique comme Eudragit® NE 30 D, dans lesquelles viennent en question pour une partie en poids de flupertine (rapporté à la base), de 0,001 à 20 parties de composant retard et la vitesse de libération de la flupertine s'élève entre 5 et 300 mg par heure, déterminée selon la méthode de l'USP XXII avec l'appareillage 2 dans une solution d'essai aqueuse de pH 1,0 et/ou de pH 6,8.

3. Préparations médicamenteuses selon la revendication 1 ou la revendication 2,
**caractérisées en ce que**
les formes d'administration pour l'utilisation orale renferment de 50 à 600 mg, pour l'utilisation parentérale de 50 à 500 mg et pour l'utilisation dermique de 5 à 5000 mg, de principe actif flupertine.

4. Préparations médicamenteuses selon la revendication 1,
**caractérisées en ce qu'**
on inclut de préférence 1 partie en poids de principe actif flupertine dans 0,5 à 5 parties en poids de composant retard.

5. Préparations médicamenteuses selon la revendication 2,
**caractérisées en ce qu'**
on enrobe de préférence 1 partie en poids de principe actif, la flupertine avec 0,01 à 5 parties en poids de composant retard.

6. Préparations médicamenteuses selon la revendication 1,
**caractérisées en ce que**
comme graisses digestibles on met en oeuvre de l'huile d'arachide ou de l'huile de ricin hydrogénée.

7. Préparations médicamenteuses selon la revendication 1,
**caractérisées en ce que**
comme graisses non digestibles on met en oeuvre des esters d'acide gras aliphatiques, saturés ou non saturés, ayant de 2 à 22 atomes de carbone, avec des alcools aliphatiques unifonctionnels, ayant de 1 à 20 atomes de carbone, de la cire de carnauba, de la cire d'abeilles, ou des alcools gras ayant de 8 à 30 atomes de carbone.

8. Préparations médicamenteuses selon la revendication 1,
**caractérisées en ce que**
comme polymères on met en oeuvre de l'alcool polyvinylique, de l'acide polyacrylique, des produits de polymérisation anioniques à partir d'acide méthacrylique et d'esters d'acide méthacrylique (Eudragit® L, Eudragit® S), des produits de copolymérisation d'ester d'acide acrylique et d'ester d'acide méthacrylique avec le méthacrylate de triméthylammonium (Eudragit® RL, Eudragit® RS), des produits de copolymérisation à partir d'ester éthylique d'acide acrylique et d'ester méthylique d'acide méthacrylique, (Eudragit® NE 30 D) ainsi que des produits de copolymérisation à base d'acide acrylique, d'acide méthacrylique ainsi que de leurs esters (rapport des groupes carboxyle libres aux groupes ester 1:1) (Eudragit® L 30 D).

9. Préparations médicamenteuses selon la revendication 1,
**caractérisées en ce que**
comme substances gonflantes on met en oeuvre des méthylcelluloses, des hydroxypropylcelluloses, des hydroxypropylméthylcelluloses, de l'acide alginique et ses sels, également des mélanges à base d'alginate de sodium et de sels de calcium, des amidons, des carboxyméthylamidons, des carboxyméthylcelluloses et leurs sels, du galactomannane, de la gomme arabique, de la gomme Karaya, de la gomme Ghatti, de l'agaragar, du carragène, de la gomme xanthane, de la gomme guar et ses dérivés, de la farine de graines de caroube, de l'alginate de propylèneglycol, de la pectine et de la gomme adragante.

10. Procédé de fabrication de préparations médicamenteuses ayant une libération contrôlée du principe actif, contenant de la flupertine ou un de ses sels physiologiquement compatibles, conformément à la revendication 1,
**caractérisé en ce qu'**
on transforme une partie en poids de flupertine (rapporté à la base) avec de 0,001 à 20 parties d'un composant retard et le cas échéant en ajoutant des substances de support, des celluloses, des agents diluants, des agents liants, des agents de démoulage, des agents d'éclatement, et/ou des agents de conservation, des agents stabilisants, des retardateurs de sédimentation ou des agents anti-oxydants et on ajuste une vitesse de libération de la flupertine, comprise entre 5 et 300 mg par heure, déterminée selon la méthode de l'USP XXII avec l'appareillage 2, dans une solution d'essai aqueuse de pH 1,0 et/ou de pH 6,8.

11. Procédé de fabrication de préparations médicamenteuses ayant une libération contrôlée du principe actif, contenant de la flupertine ou un de ses sels physiologiquement compatibles, conformément à la revendication 2,
**caractérisé en ce qu'**
on transforme une partie en poids de flupertine (rapporté à la base) avec de 0,001 à 20 parties d'un composant retard et le cas échéant en ajoutant des agents plastifiants, des matières solides comme le talc et/ou le stéarate de magnésium, des polyéthylèneglycols, de la polyvinylpyrrolidone et d'acétate de polyvinyle, de l'hydroxypropylcellulose, de l'hydroxypropylméthylcellulose, et/ou des acides organiques, et on règle une vitesse de libération de la flupertine entre 5 et 300 mg par heure, déterminée selon la méthode de l'USP XXII avec l'appareillage 2 dans une solution d'essai aqueuse de pH 1,0 et/ou le pH 6,8.

12. Procédé de fabrication de préparations médicamenteuses selon la revendication 10 ou 11,
**caractérisé en ce que**
le principe actif est enrobé avec un ou plusieurs composants retard ou inclus dans un ou plusieurs composants retard.

13. Préparations médicamenteuses contenant de la flupertine ou un de ses sels physiologiquement compatibles,
**caractérisées en ce qu'**
à côté d'une partie ayant une libération contrôlée du principe actif, accessible par le procédé conforme à la revendication 10 ou la revendication 11, une partie ayant une libération immédiate du principe actif est contenue, pour laquelle le rapport dans la teneur du principe actifflupertine entre la partie ayant une libération contrôlée du principe actif et la partie ayant une libération immédiate du principe actif, se situe entre 1:2 et 9:1.
